# EUROPEAN PATENT APPLICATION

(11) **EP 4 364 663 A1**
(43) Date of publication of application: **08.05.2024**
(21) Application number: 22205894.3
(22) Date of filing: 07.11.2022
(51) Int. Cl.: A61B 5/256, A61B 5/27

(54) **SMART SENSING TEXTILE**

(71) Applicant: Nederlandse Organisatie voor toegepast-natuurwetenschappelijk Onderzoek TNO, 2595 DA 's-Gravenhage (NL)
(72) Inventor: KJELLANDER, Birgitta Katarina Charlotte, 2595 DA 's-Gravenhage (NL); DASSEN, Maxime Pauline Aline, 2595 DA 's-Gravenhage (NL)
(74) Representative: V.O.

(57) **Abstract**

The present disclosure pertains to a wearable electronics product (1), a use method, and a kit of parts.

The product comprises; a stretchable textile (2) comprising at least one skin contact electrode (5-1) for interfacing with a skin of a wearer. The skin contact electrode comprises an electrically conductive stretchable textile (15-1) that is bordered by an electrically insulating stretchable textile (17); and a flexible patch (4) supporting electronic components (42) connected to a flexible contact terminal (7-1) of the flexible patch (4). The wearable electronics product comprises a securing element (9) that reversibly mounts the flexible patch (4) along an outward side (23) of the stretchable textile and applies contact pressure F at overlaps between the skin contact electrode and the contact terminal, thereby maintaining an electrical interconnect between the skin contact electrode and the contact terminal (7-1) at least during wear.

## Description

### TECHNICAL FIELD AND BACKGROUND

The present disclosure relates to wearable electronics products, a method of using the wearable electronics product, a kit, and individual parts for assembling the wearable electronics product.

Measuring electrical signals (e.g. bio signals) or applying electrical signals to the skin of a subject typically involves adhering electrodes (e.g. self-adhering electrodes) to the skin at one or more, typically a plurality, of relevant positions and connecting relevant electronic device to said electrodes. When monitoring body functions, e.g. ECG signals, respiration (as e.g. bio-impedance), or stress levels (as e.g. skin conductance), it is important to accurately position skin-contact sensors at desired positions on the body. The arrangement of these positions in relation to one another differs from person to person, e.g. body to body type, and/or between applications.

WO2015067626 discloses a textile device having integrated therein a first and a second electrically conductive portion. The device also comprises an electrical element having a first contact pad which is electrically connected to the first textile portion and a second contact pad which is electrically connected to the second textile portion, wherein the first and second textile portions are adapted to supply the electrical element with electrical power.

WO2001095752 discloses a garment comprising a fabric shell hosting electrical conductor connectable to at least one of a plurality of electronic devices housed in respective pockets of the fabric shell.

WO2006035385 discloses a modular wearable circuit, comprising any number of garments that can cooperate as complementary garments in a set. Each garment comprises integrated conductive networks and electronic components.

US2019297961 discloses a textile product with a built in skin contact element and/or a built in electrode which can serve as a sensor for measuring body functions. Specifically, connection technology between the skin contact element and electronic measuring and evaluation unit is disclosed.

WO2020099906 discloses a textile based ECG sensor system, comprising a non-conductive substrate with textile based sensors positioned on a first side thereof, and insulated by a second side thereof. Each of the textile based sensors includes conductive fibers interlaced with one another. Conductive traces are connected to the each of the textile-based sensors for sending and receiving signals from an electronic controller.

While one or more of the products describe above may mitigate disadvantages associated to conventional adhering of electrodes to the skin the skin of a subject there remains a need for textile products enabling reliable measurement/application of electrical signals to/from the skin of a person. In particular there remains a need for products offering one or more of improved robustness, e.g. in terms of washability or reusability, improved wear comport, and or products with improved functionality, e.g. in terms of personalizability for specific body shapes or body types.

### SUMMARY

The present disclosure aims to mitigate disadvantages of known products and/or provision of products addressing the above needs. As will be clear from the disclosure herein below the present disclosure relate to textile products offering increased robustness during use, improved reusability, and/or that are adaptable for various body types and different applications.

In particular the present disclosure provides a smart textile product with improved robustness during normal wear and tear, such as washing, stretching, folding, bending and handling during its lifetime, while at least maintaining accuracy of electrical signal transduction to/from skin.

In line with an aspect of the invention there is provided a wearable electronics product. The product comprises a stretchable textile product, a flexible patch and a securing element. The stretchable textile product is configured for conformable wearing around a body part of a wearer. The stretchable textile product comprising at least one skin contact electrode. The skin contact electrode is bordered by an electrically insulating stretchable textile to electrically insulate skin contact electrode from any adjacent skin contact electrodes. The skin contact electrode is comprised of an electrically conductive composition, typically en electrically conductive stretchable textile. The skin contact electrode comprises a first face extending along an inward side of the textile product, and a second face extending along an outward side of the textile product. This skin contact electrode, e.g. the conductive textile, provides an electrically conductive pathway between opposing faces of the textile product, e.g. between the opposing first and second faces (a skin facing side and an opposing outward face) of the skin contact electrode. As such, at least during wear the skin contact electrode can be understood as forming an element for interfacing with the skin of the body/wearer.

In a preferred embodiment the stretchable textile product is a garment e.g. a base layer garment for wearing directly on a skin of a wearer. Alternatively, the textile product can be intermediary product, e.g. a textile sheet, a bandage, or inlay, e.g. for directly contacting a skin of a wearer wear, e.g. by positioning the sheet under a base layer garment.

The wearable electronics product, at least during wear, further comprises one or more electronic components. These components can include components providing functionality to the device, including but not limited to one or more of: sensor(s), actuator(s), processor(s), and/or elements for powering the component(s) (e.g. battery). The components can be provided on one or more PCBs. The PCB can contain wireless communication components (I/O devices), e.g. bluethooth or wifi modules.

The one or more electronic components are typically supported by one or more flexible patches. The one or more electronic components are generally operably connected to one or more flexible contact terminal of the flexible patch, e.g. by flexible wiring, such as a conductive track printed or otherwise formed on the patch.

The wearable electronics product further comprises a securing element configured to reversibly mount (mount and unmount) the flexible patch along an outward side of the stretchable textile product. The mount is advantageously configured to apply a contact pressure at a position of an overlap between an outward face of the skin contact electrode and the contact terminal. Thus forming an electrical interconnect between the skin contact electrode and the contact terminal at least during wear. In a preferred embodiment even the securing element can be provided in the form of a replaceable consumable.

The stretchable textile product, in particular the electrically conductive stretchable textile and the electrically insulating stretchable textile portions thereof enable a snug fit around a relevant body part of the wearer, mitigating potential relative movement of the skin contact electrode relative to the skin, even during movement/motion of the wearer. At the same time the securing element can advantageously fix a relative position between the electrically insulating stretchable textile and the contact terminal of the supported patch, thus realizing a stable reliable electrical interconnect between the one more electronic component and the skin.

The electrically conductive stretchable textile can comprise one or more metal-based yarn. Advantageously, the conductive yarn can be woven with non-conductive yarn to respectively form conductive and non-conductive portions of the textile product. Preferably the electrically conductive stretchable textile comprises metallic or metal plated conductive fibers or yarns. Silver, having excellent antibacterial properties and skin compatibility be a particularly material.. Alternatively, or in addition, the skin contact electrode may comprise a conductive coating, e.g. a flexible polymer-based matrix or other composition with conductive fillers as known in the field. A flexible conductive coating, e.g. a silicon-based coating with conductive fillers can be particularly advantageous at one or more of a skin facing and outward facing sides of the skin contact electrode to respectively reduce a respective contact resistance. In some embodiments, the skin contact electrodes can provide anisotropic electrical conductive properties isolated portions or islands of conductive stretchable textile, integrally formed e.g. stitched, woven, glued, etc. with a layer of non-conductive, insulating stretchable textile.

Supporting the electronic components along an outside face of the textile improves a wear comfort and minimizes disturbances to a conformal contact between skin and the skin contact electrode during wear. An advantage gained by reversibly securing the flexible patch to the outward side of the textile product is that only the textile product needs comply with bio-, specifically skin compatibility. Other components, including the flexible patch, its contact terminals, and/or wiring, can, but not need to, comply with bio-, specifically skin-, compatibility because the configuration as disclosed herein advantageously avoids a direct skin-contact of these parts. This allows more flexibility in selection of materials/compositions for these parts.

By providing a securing element that is reversibly mountable to the outward side of the stretchable textile product, the securing element can be used to reversibly mount the patch to the textile product. Reversibly mounting can be understood as removably, detachably, non-permanently or even repeatedly mounting, fixing, securing or attaching the securing element and/or patch to the outward side of the textile product.

Providing the wearable electronics product with a securing element for reversibly mounting the flexible patch along an outward side of the stretchable textile product allows a user to remove the flexible patch with its contact terminals and circuitry from the textile product when desired. The flexible patch can thus be removed from the textile product before washing the textile product. Providing a securing element for reversibly securing the flexible patch to the textile product additionally yields a robust, flexible wearable electronics product, an interchangeability between various textile products and flexible patches along with improved washability of the textile products.

When operably connected to skin contact electrodes as described herein, the flexible patch can have various applications. The skin contact electrodes can be used as input or output electrodes. In an scenario wherein the flexible patch operates as a sensor, e.g. to measure a response (surface potential) from the skin of a wearer, the skin contact electrode can act as an input electrode, delivering an input to the flexible patch, from the skin of a user. In an alternative scenario wherein the flexible patch operates as an actuator, e.g. applying a potential difference to the skin of a wearer for electrostimulation, the skin contact electrode can act as an output electrode, delivering an output from the flexible patch to the skin of a wearer.

As provided by the present disclosure the flexible patch can be removed from the textile device to be reattached at a different functional target area or region of interest or portion of the textile product. The wearable electronics product can be disassembled, and reassembled for a different use, including use on a different body part of the same or even a different wearer. As will become clear from the specification the modularity of the system can further enable personally tailored and/or more accurate (re)positioning of the flexible patch along regions of interest. For example, the flexible patch can first be assembled onto an area of a textile product corresponding to a wearers heart placement in order to measure an ECG signal. Subsequently, the flexible patch can be removed and reassembled (e.g. with a different or additional flexible patch) onto a different area of the textile product, e.g. in order to monitor or electrically stimulate muscles.

Alternatively, or in addition, the wearable electronics product can be disassembled, and reassembled for use on a different wearer, with a different body configuration to a previous wearer. An advantage gained by reversibly securing the flexible patch to the textile product lies therein that it allows the same wearable electronics product to be reusable for a plurality of wearers, with varying body shapes/sizes/physiology. Additionally, the same flexible patch can also be compatible with a plurality of different textile products. The reusability and compatibility of the flexible patch in multiple different applications, body parts, textile products and body types improves the customizability and personalizability of the wearable electronics product.

The skin contact electrodes and surrounding textile are preferably durably joined to form an integral part, single sheet, of the stretchable textile product, e.g. by being stitched, glued, woven or kitted, to a single element etc. Preferably, the electrically conductive stretchable textile and the electrically insulating stretchable textile are be seamlessly formed as a continuous textile, preferably a woven, nonwoven or knitted textile. Seamlessly joining the electrically conductive portion and the electrically insulating portion of the textile product as a single, continuous textile sheet, preferably a woven, nonwoven or knitted textile, reduces a number of potential pressure points during wear, thus improving wear comfort, securing element. A seamless textile product also allows for more uniform stretchability over the textile product, as rigid or less stretchable seams are avoided. Preferably, at least the electrically insulating stretchable textile, is a so-called 4D stretchable fabric. A 4D-textile, e.g. a 4D-KNIT can be completely variable in shape, positioning and height. More preferably both the electrically insulating stretchable textile and the electrically conductive stretchable textile are 4D stretchable.

In a preferred embodiment, the flexible patch comprises a plurality of contact terminals and the stretchable textile product comprises at least a corresponding number of skin contact electrodes, whereby the skin contact electrodes and contact terminals are mutually configured to provide electrical interconnects between the respective contact terminals and separate ones of the skin contact electrodes.

It will be understood that the number of skin contact electrode and contact terminals can depend on an intended application. The patch can be a patch as known in the field, e.g. a patch be configured to measure a signal, such as a potential difference, between the two contact terminals, e.g. for ECG, EEG or EHG measurements. For example, for ECG-measurements of configuration with at least three skin (e.g. 3, 5, or 7), contact electrodes (and corresponding contact terminals may be provided. In other embodiments, the flexible patch comprises four or more contact terminals. For some applications, e.g. subcutaneous muscle stimulation, a different number of skin contact electrodes may suffice, e.g. one. It will be understood that the wearable electronics product may provide a plurality of individualize patches jointly providing a single or even a plurality of functionalities as described herein.

In a yet another or further embodiment the stretchable textile product comprises a plurality of the skin contact electrodes each electrically separated or isolated from adjacent ones and arranged in an array. The array defining a functional area of the stretchable textile product, whereby the skin contact electrodes are dimensioned and interspaced such that the contact terminal overlaps with the skin contact electrode, and any additional contact terminals overlap with mutually exclusive ones of the skin contact electrodes. Advantageously, the functional area of the textile product covered by the conductive array, can be larger than a target measurement area or region of interest along the skin of the body part known to correspond to a target bio signal of the body part. Providing a plurality of skin contact electrodes covering a functional sensing and/or stimulus area larger than a region of interest of a body part allows the textile product to be compatible with different body types of different users.

Further, flexible patches or flexible patches to be functionally connected to the textile product can have varying dimensions and/or differing placement and orientation of contact terminals. Therefore, dimensioning the functional area larger than a target measurement area or region of interest allows the textile product to be compatible with many different flexible patches or flexible patches, improving customizability and personalizability of the wearable electronics product.

In some preferred embodiments the flexible patch comprises a flexible carrier, supporting or comprising one or more contact terminal as specified herein. The flexible patch can comprise or support thereon, one or more electronic components. Alternatively, or in addition, the flexible patch may comprise circuitry to operably connect the one or more contact terminals to the one or more electronic components. Advantageously, the flexible patch can conform to a body part of a wearer to which it is applied, improving overall comfort of the wearable electronics product. Advantageously the but not need to be stretchable. The patch can be a disposable patch, such as a single use patch. In more preferred embodiments the patch is a reusable patch. The circuitry, and optionally the contact terminals, can comprise printed circuitry of flexible or stretchable ink, applied onto the flexible carrier. The contact terminals and/or the flexible patch can comprise a patterned shape, having holes or discontinuities e.g. perforated area, spiral shape, grid shaped, etc. for increased flexibility and breathability. The electronic component may be one or more selected from the list of a PCB, battery, electronical drive actuator, vibration motor, LED, sensor, and/or photodiode. Advantageously the patch does not need to be washable, since it can be removed before washing. The patch can be a disposable or reusable patch. Preferably the patch and/or the textile product is reusable. For scalability, the patch may be produced in a similar way to a health patch i.e. the patch may comprise printed circuitry of flexible or stretchable inks, preferably on plastic foils, e.g. TPU or PET.

Advantageously, the electronic component and any circuitry associated therewith can be entirely disposed on the flexible patch. By limiting all wiring and circuitry to the removable patch, an advantageous modular design is achieved. By having no wiring and/or circuitry comprised in the textile product, all wiring and/or circuitry can be removed from the textile product with the patch, allowing the textile product to be readily washed without damaging any delicate or water incompatible circuitry. Having no electronic components or circuitry disposed between the skin and the textile product, limits wear due to rubbing and improves the lifetime of the textile product. Of course the readout does not need to be a printed electronics patch, mixed solutions or wire are also envisioned.

In a preferred embodiment the securing element overlaps the flexible patch and exerts a securing pressure normal to the textile product at least at each of the electrical interconnects. The securing element can for example be a pocket or a strap. Advantageously, the securing element can also be a modular element that can be reversibly attachable to the textile product in accordance with a position of the flexible patch. Providing a reversibly attachable securing element, increases the flexibility as to positing of the flexible patch, and accordingly improves the versatility and customizability of the wearable electronics product as a whole, making it more personalizable.

By providing a securing element that covers, preferably completely covers, the flexible patch, the flexible patch can be shielded from external contact, protecting the flexible patch, the components and/or wiring from external interference, improving sensing robustness and a general visual aesthetic of the wearable electronics product, as the flexible patch can be hidden from view.

Means to reversibly attach the securing element to the textile product can include snap-connectors, adhesives (e.g. tape), clamps, non-permanent or detachable adhesives, magnets or hook and loop attachments. Alternatively, or in addition, said means may be understood as, forming the securing element or at least in part contributing to securing the flexible patch to the stretchable textile product.

In line with a further aspect of the invention there is provided a method of using the wearable electronics product described herein, the method including a step of fitting the stretchable textile product to a body part of a subject, such that the textile product conformably follows the curvature of the body part such that the functional area of the stretchable textile product, as defined by the skin contact electrode and one or more of the further skin contact electrodes (if present) of the stretchable textile product covers a region of interest of the body part. The region of interest may be an area along the wearer's skin known to correspond with positions for receiving a target bio signal of the body part and/or known to correspond with positions for applying an electrical stimulus to the body part.

The method typically further includes a further step of mounting the flexible patch along an outward side of the stretchable textile product, within a perimeter of the region of interest, thereby forming an electrical interconnect between respective contact terminals and separate ones of the skin contact electrodes. The patch is preferably positioned at the relevant site while the textile product is worn by the user. The patch is secured at the relevant position by the securing means. In some embodiments, e.g. when the securing means is pre-attached a pocket or strap the patch can eb simply inserted, e.g. slid between the exterior face of the textile and the securing means.

In some embodiments, the method can include yet a further step of removably attaching the securing element, preferably after fitting, to the outside side of the textile product such that the securing element covers the region of interest.

The method can comprise an additional step of identifying a region of interest within the functional area of the textile product. For example, if the target bio signal is an ECG signal, the region of interest can correspond with a position close to the heart of a wearer. Mounting the flexible patch while the textile product is being worn improves the accuracy with which a user can position the flexible patch, as the region of interest can be identified visually.

Optionally, an electrically conductive paste, preferably a non-permanent conductive adhesive, can be provided between one or more of the skin contact electrodes and one or more of the contact terminals, thereby improving the robustness and integrity of the electrical connections.

In a preferred method, mounting, preferably reversable mounting or attaching, of the securing element to the textile product can include providing an adhesive along at least an edge portion of the securing element prior to contacting the securing element to the textile product. The adhesive is preferably a non-permanent adhesive, e.g. an removable adhesive tape or an adhesive releasable during washing.

Mounting the securing element to the textile product preferably comprises contacting the pocket to the textile product with the pocket and/or the textile product in a fitted or similar pre-stretched condition. By mounting the securing element, such as a pocket or strap, to the outward side of the stretchable textile product while the textile product is being worn by the wearer allows the user to more easily mount the securing element in such a manner that the resulting securing pressure maintains an electrical connection between the skin, skin contact electrodes and contact terminals. Mounting the flexible patch to an outward side of the stretchable textile product after fitting the textile product to a body part of a wearer allows the full range of stretchability of the textile product to be utilized, without being limited by rigid or less flexible electronic components. Having the flexible patch detached from the flexible textile during fitting mitigates damage to the patch due to stretching, bending, and handling.

It will be appreciated that one or more, or even all, of the steps of fitting the textile product, mounting or position of the flexible patch, removably attaching the securing element, and/or identifying the relevant position, may be performed by the user. Alternatively, or in addition, part or all of the steps may be performed by, or under the guidance of, a medical professional. A set of instructions may be provided to guide the user/professional.

Depending on the application the method may further include a step of receiving a bio signal, preferably a vital sign, or applying an electrical stimulus. By coupling the patch to an output device, the received bio signal may be output in such a manner that it can be interpreted by a user, e.g. a medical practitioner or even a wearer.

It will be appreciated that an embodiment of the textile product having an array of skin contact electrodes as disclosed herein is particularly preferred for obtaining a modular product.

Preferably, the textile product is dimensioned to provide a tight fitting so that contact between the wearer's skin and the skin contact electrodes are maintained, improving the accuracy of signals measured by the flexible patch. Tight fitting in the context of the textile product means that the textile product exerts a normal force on the skin surface of the wearer as a result of tensile forces present in the stretchable textile when it is in a stretched condition around the body part.

In line with another or further aspect of the invention there is provided a kit of parts for assembling the wearable electronics product as disclosed herein. The comprising the stretchable textile product, the patch, the securing element and optional instructions for using the wearable electronics product. The instructions can list the steps of the method on how to use the kit, describing steps of fitting the stretchable textile product to a body part, identifying a region of interest, reversibly mounting the flexible patch to an outward side of the textile product, and/or reversibly attaching the securing element to an outward side of the textile product. Optionally, further instructions can include steps of measuring a bio signal, such as a vital sign. The kit can further include a data read-out device, and/or a data storage device, and/or a data transmission device. Depending on at least the electronic components provided, wearable electronics product can advantageously be configured as a wearable sensing product, and/or a wearable actuating product.

In line with yet a further aspect of the invention there is provided a stretchable textile product, preferably a garment, configured to and evidently suitable for forming the wearable electronics in combination with an flexible patch and a securing element, preferably the flexible patch and the pocket or strap as specified herein. The garment can e.g. be a garment selected from: a base layer garment such as a shirt, leggings, short, bra, belt, sleeve, chest band, waist band, arm band, leg band, head band, sock, bandage, and the like.

The products, kits and/or devices described herein can be used in medical applications, e.g. for measuring and/or monitoring vital signs, ECG, bio-impedance, breathing, electrodermal conductance, muscle activity (EHG), EEG, for diagnostic purposes, for physiotherapy, etc. The products, kits and/or devices described herein can be specifically useful in medical treatment facilities such as in hospitals, clinics, physical rehabilitation centers, emergency response units, etc. Further, the products, kits and/or devices described herein can be useful for personal use, for example monitoring vitality, measuring and indicating fitness parameters during exercise, electrical muscle stimulation, etc. The wearable electronics product can be a medical device for use in a medical context, for example to prevent, diagnose, treat and/or rehabilitate illnesses, diseases and/or other conditions.

### BRIEF DESCRIPTION OF DRAWINGS

These and other features, aspects, and advantages of the apparatus, systems and methods of the present disclosure will become better understood from the following description, appended claims, and accompanying drawing wherein:
FIGURES 1A and 1B show cross-sectional views of embodiments with a flexible patch and securing element reversibly mounted to a textile product,
FIGURE 2 shows a top view of an exemplary flexible patch and securing element in a mounted configuration,
FIGURE 3A shows a flexible patch reversibly mounted to a textile product,
FIGURE 3B provides an enlarged partial view of FIG 3A,
FIGURE 3C shows a wearable electronics device in an assembled condition,
FIGURES 4A-E show different embodiments of a textile product with different arrangements of skin contact electrodes,
FIGURES 5A and 5B show embodiments of cross-section A of the textile product depicted in Fig. 4B,
FIGURE 6 shows an array of skin contact electrodes,
FIGURES 7A-C show a wearable electronics product worn by a user,
FIGURES 8A-C illustrate aspects of mounting a securing element to the textile product,
FIGURE 9 schematically illustrates a method of using a wearable electronics product, and
FIGURES 10A-F provide exemplary experimental results.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The wearable electronics product 1 will now be explained in further detail with reference to the figures.

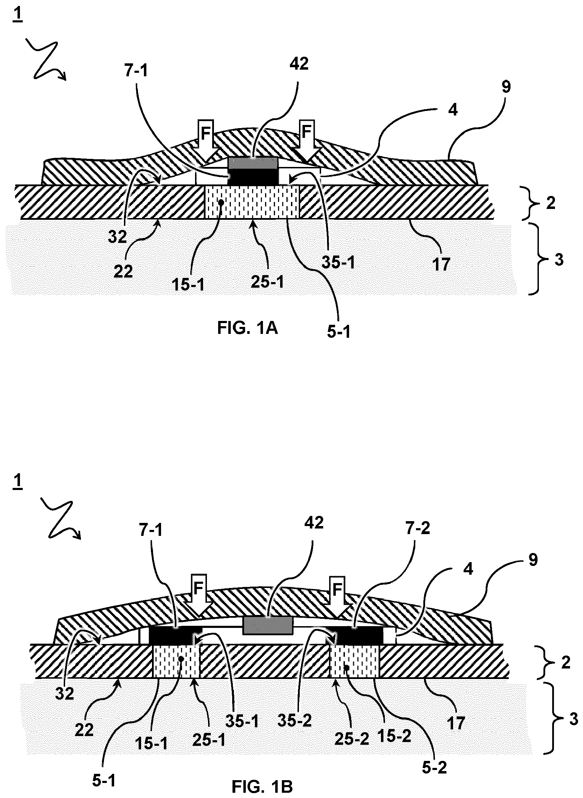

The wearable electronics product 1, as shown in the figures generally comprises a stretchable textile product 2, a flexible patch 4 and a securing element 9. The wearable electronics product 1 is flexible and stretchable for conformably following a curvature of a body part 3. As depicted in FIG 1A, the stretchable textile product 2 can comprise at least one skin contact electrode 5-1 for interfacing with a skin of the body part 3 during wear. The skin contact electrode 5-1 can comprise an electrically conductive stretchable textile 15-1 bordered by an electrically insulting stretchable textile 17.

In a preferred embodiment, the wearable electronics product comprises a flexible patch 4 supporting thereon an electronic component 42 operably connected to a flexible contact terminal 7-1 of the flexible patch 4.

The wearable electronics product 1 preferably further comprises a securing element 9 reversibly mounting the flexible patch 4 along an outward side 32 of the stretchable textile product 2 and applying a contact pressure at a position of an overlap 8-1 between an outward face 35-1 of the skin contact electrode 5-1 and the contact terminal 7-1 forming an electrical interconnect between the skin contact electrode 5-1 and the contact terminal 7-1 at least during wear.

Alternatively, or in addition, the flexible patch 4 comprises a plurality of contact terminals 7-1,7-2,7-n and the stretchable textile product 2 comprises at least a corresponding number of skin contact electrodes 5-1,5-2,5-n. The skin contact electrodes and respective contact terminals can be mutually configured to provide electrical interconnects between the respective contact terminals and separate ones of the skin contact electrodes.

One or more, preferably all, of the stretchable textile product 2, the skin contact electrodes and the electrically insulting stretchable textile 17 may comprise a four directional stretchable textile. It will be understood that a four directional stretchable means that the textile can be stretched and recover an initial shape both crosswise and lengthwise.

As depicted in FIG 1B, the stretchable textile product 2 can comprise at least a first and a second skin contact electrode 5-1, 5-2. Each skin contact electrode can be formed by a flexible electrically conductive portion 15-1,15-2 of the textile product 2, and bordered by an electrically insulting portion 17 of the stretchable textile product.

FIG 1B shows each skin contact electrode formed of an electrically conductive stretchable textile having an inward face 25-1,25-2 extending along an inward side 22 of the textile product, and an outward face 35-1,35-2 extending along an outward side 32 of the textile product. As depicted, during wear, the inward face 25-1,25-2 of the skin contact electrode faces towards, and preferably directly or indirectly contacts, the skin of the body part 3. It will be appreciated an electrically conductive coating (e.g. a silicon-based coating 15c1) can be applied to an inward face 25-1,25-2 of a skin contact electrode, e.g. for transmitting electrical current/charges between the skin and the skin contact electrodes. A similar or further coating 15c2 provided to an outward textile side may reduce contact resistance with the contact terminal. The outward face 35-1,35-2 of the skin contact electrode faces towards, and preferably directly or indirectly contacts, the contact terminal 7-1,7-2 of the patch 4. Optionally, an electrically conductive paste, preferably a non-permanent conductive adhesive, can be provided between the outward face 35-1,35-2 and a contact terminal 7-1,7-2 of the flexible patch, thereby improving the robustness and integrity of the electrical and mechanical interconnects.

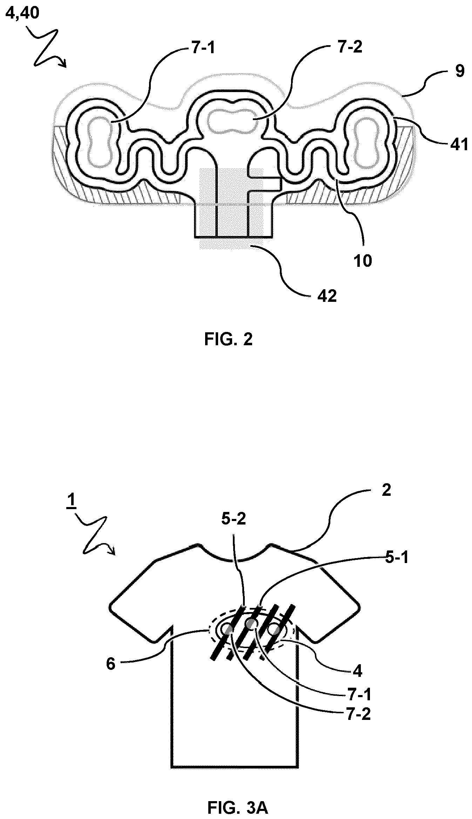

The flexible patch 4 generally comprises at least a first and a second contact terminal 7-1,7-2 and circuitry 10 operably connected thereto, as can be seen in FIG 2. The flexible patch 4 may comprise a flexible substrate, supporting thereon one or more electronic components 42 and/or circuitry 10. The one or more electronic components 42 may be one or more selected from the list of a PCG, battery, electronical drive actuator, vibration motor, LED, sensor, and/or photodiode.

In a specifically preferred embodiment the stretchable textile product 2 comprises a plurality of the skin contact electrodes 5-1,5-2,5-n each electrically separated from adjacent ones and arranged in an array defining a functional area of the stretchable textile product 2, whereby the skin contact electrodes are dimensioned and interspaced such that the contact terminal 7-1 overlaps with the skin contact electrode 5-1, and any additional contact terminals 7-1,7-2,7-n overlap with mutually exclusive ones of the skin contact electrodes 5-1,5-2,5-n.

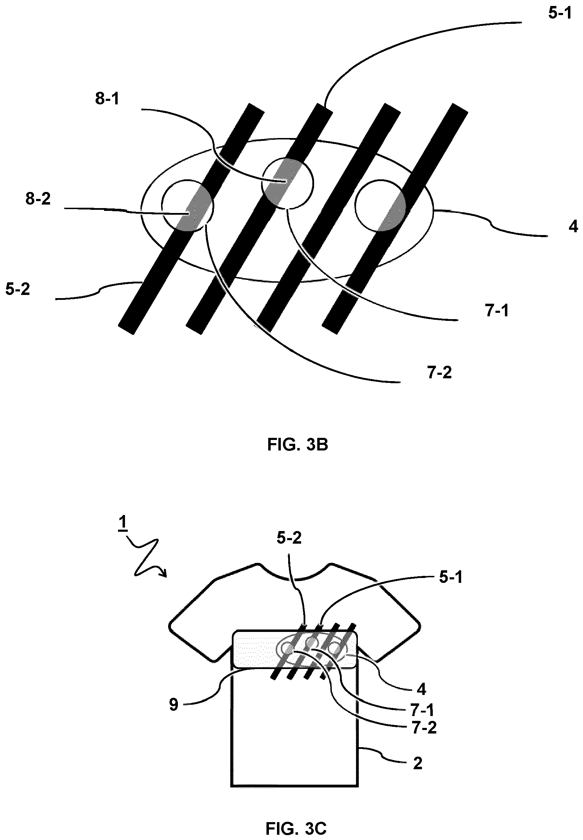

FIG 3A shows an embodiment of a wearable electronics device 1 wherein the flexible patch 4 is reversibly mounted to the stretchable textile product 2, at a position corresponding to a region of interest 6 within a functional area of the textile product, the region of interest known to correspond with positions for receiving a target bio signal of the body part and/or known to correspond with positions for applying an electrical stimulus to the body part. FIG 3B shows the skin contact electrodes 5-1,5-2 and contact terminals 7-1,7-2 mutually dimensioned and aligned to provide a first overlap 8-1 between the first skin contact electrode 5-1 and the first and the second contact terminal 7-1, and a mutually exclusive second overlap 8-2 between the second skin contact electrode 5-2 and the second contact terminal 7-2, to form a first and a second electrical interconnect. It will be appreciated that the relative dimensions and positioning of the skin contact electrodes and the contact terminals, can ensure that each skin contact electrode contacts at most one contact terminal, when the patch is mounted to the textile product within a target measurement area.

FIG 3C shows an embodiment of a wearable electronics product 1 wherein the securing element 9 is reversibly mounted to the stretchable textile product 2. The securing element 9 can be a flexible, conformable pocket or strap. The securing element preferably has a stiffness that is higher than a stiffness of the textile product in preferably one direction, optionally in two directions. Preferably the securing element 9 has a Young's modulus that is higher than a Young's modulus of the textile product 2. By providing a securing element 9 with a higher stiffness than the textile product, having low or optionally no stretch, preferably in a direction along the length of the circuitry, damage to the circuitry due to stretching is mitigated. When the wearable electronics product is in use, a securing pressure F exerted by the securing element maintains a relative position between the contact terminals 7-1,7-2 and the skin contact electrodes 5-1,5-2, thereby maintaining an electrical connection. The securing pressure F exerted by the securing element 9 further maintains a relative position between the skin contact electrodes 5-1,5-2 and the wearer's skin, thereby maintaining an electrical connection with the skin. By mitigating relative motion between the skin contact electrodes and contact terminals, and/or between the skin contact electrodes and the wearer's skin, the signal quality and stability as well as the sensing robustness is improved. The securing element 9 is preferably reversibly mountable, i.e. removably attachable to the outward side 23 of the stretchable textile product 2.

In order to increase support, i.e. normal force of securing pressure F, for securing the flexible patch 4 to the textile product 2, and increase support for maintaining electrical interconnects between the skin contact electrodes and contact terminals, the material used for a securing element, e.g. in the form of a pocket or strap, is preferably less stretchable than the material used to form the textile product. Securing element materials, especially materials for forming a pocket as described herein, ideally have an elasticity which is in a range of 3 to 8 times lower than that of the material used for forming a textile product, preferably in a range of 4 to 7 times lower. For example, in an exemplary embodiment the elasticity is about 5 times (±10%) lower than that of the material used for forming a textile product.

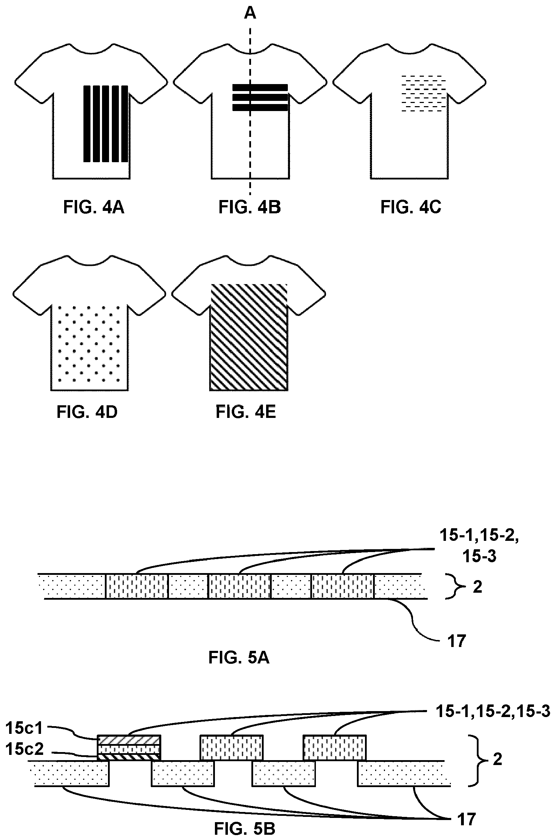

FIG 4A-4E depict non-exhaustive examples of alternative embodiments of a plurality of skin contact electrodes 5-1,5-2,5-n arranged in an array spanning a functional area of the textile product 2. By dimensioning the functional area covered by the array to be larger than a region of interest area along the skin of the body part known to correspond to a target bio signal of the body part, it allows the same stretchable textile product to be compatible with a plurality of wearers, with varying body shapes/sizes/physiology.

As shown is FIG 5A and 5B, the stretchable textile product 2 can comprise conductive 15-1,15-2,15-3 and non-conductive 17 textile portions. In a preferred embodiment the electrically conductive stretchable textile 15-1 and the electrically insulting stretchable textile 17 can be seamlessly formed as a continuous textile, preferably a woven, nonwoven or knitted textile. FIG 5A shows the conductive and non-conductive textile portions seamlessly formed as a single, continuous textile sheet. By seamlessly forming the electrically conductive portion 15-1,15-2,15-3 and the electrically insulting portion 17 of the textile product as a single textile product 2, preferably a woven, nonwoven or knitted textile, the robustness and durability of the textile product 2 can be improved. Integrally forming or embedding the skin contact electrodes in the stretchable textile product by integrally forming the skin contact electrodes with the stretchable textile product improves robustness and durability of the textile product, against normal wear and tear, such as washing, stretching, folding, bending and handling during its lifetime. FIG 5B shows an alternative embodiment wherein the conductive and non-conductive textile portions forms a multiple layer textile product. The conductive and non-conductive textile portions can be bonded, attached or adhered to adjacent portions for example by stitching or gluing.

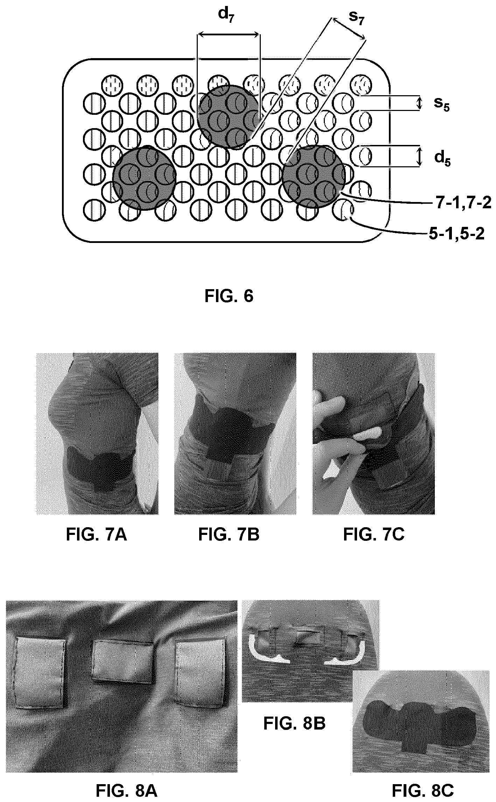

FIG 6 shows a plurality of skin contact electrodes 5-1,5-2,5-n arranged in an array along a textile product 2, and a plurality of contact terminals 7-1,7-2,7-n, of a patch 4 mounted onto the textile.

Preferably, a maximum cross sectional dimension of each the skin contact electrodes d5 and a maximum interspacing s5 between the skin contact electrodes comprised in the array are each smaller than a maximum cross-sectional dimension of the contact terminal d7 and a minimum interspacing s7 between adjacent contact terminals, if present, thereby enabling a user to functionally assemble the patch 4, and accordingly the contact terminals 7-1,7-2,7-n, at any position, and/or in any orientation within the functional area of the textile product 2. It will be appreciated that functionally assembling the flexible patch comprises mounting the patch onto the textile product such that each contact terminal of the electronic device is in contact with at least one skin contact electrode in the array, thereby forming an electrical interconnect. The pattern yielded by the arrangement of skin contact electrodes in the array described above, provides a textile product with increased customizability and ease of use. The specified relative cross sectional dimensions and interspacing of adjacent skin contact electrodes and that of adjacent contact terminals allow each contact terminal to be in contact with at least one skin contact electrode and eliminates forming of short circuits between adjacent contact terminals, regardless of the orientation, position or placement of the electronic device within the functional area of the textile product.

In a preferred embodiment, a cross-sectional dimension d5 of the skin contact electrodes is in a range of 1-100 mm, preferably in a range of 5-20 mm. A cross-sectional dimension d7 of the contact terminals can be equal or larger than the cross-sectional dimension d5 of the skin contact electrodes, such that the contact terminals can completely cover a corresponding skin contact electrode, further mitigating short circuit between skin contact electrodes.

FIGs 7A-C show a wearable electronics product 1 as described herein, comprising a stretchable textile product 2, a flexible patch 4 an a securing element 9, being worn. In a preferred embodiment the stretchable textile product has an overall extensibility along two orthogonal directions of at least 25%, more preferably at least 50% from rest, and wherein the electrically conductive and the insulating stretchable textile have a Young's modulus ratio in a range between 1:20 and 20:1, preferably in a range 1:10 and 10:1 most preferably a ratio of 1:1 ± 50%. Preferably the residual stain is as low as possible, no larger than 50%, preferably ≤ 20%, more preferably ≤ 15%. The stretchable textile product preferably comprises stretchable fabrics, such as 2-way stretch fabrics capable of stretching crosswise or lengthwise, more preferably 4-way stretch fabrics capable of stretching crosswise and lengthwise. The stretchable textile product may also comprise all way stretch fabrics, capable of stretching crosswise, lengthwise and in diagonal directions. The young modulus corresponding to the electrically conductive stretchable textile can be in a range from 0.4 to 20 MPa, preferably ≤ 2 MPa, e.g. in a range from 0.4-1 MPa. The young modulus corresponding to the electrically insulating stretchable textile can be in a range from 0.2 to 10 MPa, preferably ≤ 7 MPa, e.g. in arrange from 0.4-6 MPa. In the context of the textiles discussed here, stretchable means reversibly stretchable, i.e. in the absence of a tensile force acting on the textile, the textile essentially, preferably completely, recovers an initial condition and/or shape of the textile, corresponding to a condition and/or shape of the textile before the tensile force was applied.

In order to improve comfort during wear, the elasticity and residual strain of the textiles used for the textile product and the skin contact electrodes should ideally be similar, preferably matched. Providing a product wherein the elasticity and residual strain of the textiles used for the textile product and the skin contact electrodes are similar or ideally matched can also improve quality and stability of signals measured.

Providing a stretchable, flexible, conformable textile product enables the textile product to conformably follow the curvature of a body part to which it is applied, improving the wear comfort of the textile product. Further, by providing a tight fitting textile product which conformably follows the curvature of a body part, contact between the wearer's skin and the skin contact electrodes are maintained, improving the accuracy of signals measured by the electronic device. FIGs 7A-C shows an embodiment of a textile product described herein as a tight fitting shirt conformably following the curvature of a wearer's torso.

Advantageously the electronic component 42 and any circuitry 10 associated therewith can be entirely disposed on the flexible patch 4. By limiting any wiring and circuitry 10 to the removable patch 4, an advantageous modular design is achieved. By having no wiring and/or circuitry comprised in the textile product 2, all wiring and/or circuitry can be reversibly removed from the textile product along with the patch 4, allowing the textile product to be readily washed without damaging any delicate or water incompatible circuitry. The circuitry 10, and optionally the contact terminals 7-1,7-2,7-n, can comprise printed circuitry of flexible or stretchable ink, applied onto the flexible carrier.

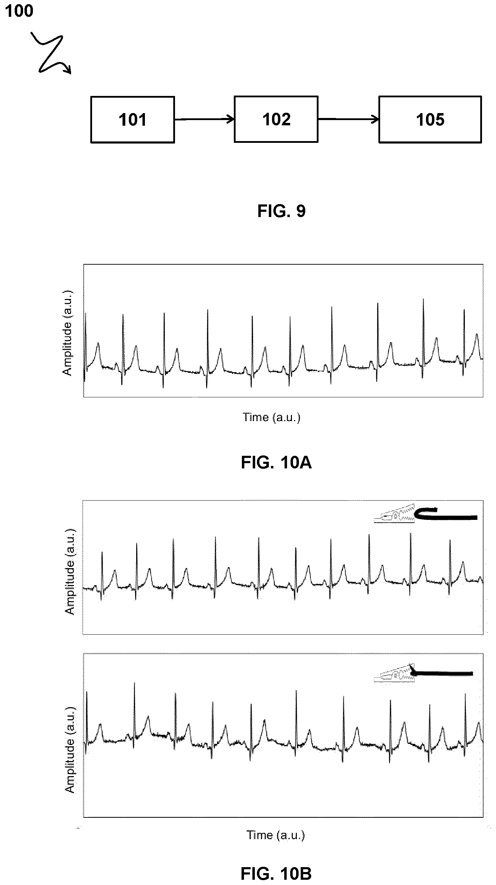

FIG 9A illustrates a method 100 of using a wearable electronics product as described herein. The method comprises the steps of fitting 101 the stretchable textile product 2 to a body part 3, such that the textile product conformably follows the curvature of the body part; and the functional area of the stretchable textile product 2 covers a region of interest 6 of the body part, and mounting 102 the flexible patch 4 along an outward side 23 of the stretchable textile product 2, within a perimeter of the region of interest, thereby forming an electrical interconnect between respective contact terminals and separate ones of the skin contact electrodes. The step of fitting the textile product preferably precedes the step of mounting the patch, e.g. inserting the patch in a pocket. Depending on whether securing element is permanently fixed, the method may include removably attaching 105 the securing element to the outside side of the textile product such that the securing element 9 covers the region of interest 6. Advantageously the securing element (e,g. pocket or strap) can be affixed after the fitting or even after positioning the patch. In some embodiments, the method comprises a step of identifying a target area on the body of the wearer. After the product is fitted and the various component are assembled the assembly can be used to measure or apply a biosignal. After use the method may comprise a step of disassembling the assembly, e.g. prior to washing the textile product. As disclosed herein the method may also comprise one or more steps of replacing the flexible patch or repositioning the flexible patch.

FIGs 8A-C show steps of removably attaching a securing element to the outside side of the textile product, with the textile product in a pre-stretched condition.

In line with an aspect of the invention, there is provided a kit of parts 200 for assembling the wearable electronics product 1, the kit comprising one or more of the stretchable textile product 2 as described herein, the flexible patch 4 as described herein and the securing element 9 as described herein. The kit 200 may further include optional instructions 201 for using the wearable electronics product according to the specified method.

In line with an aspect of the invention, there is provided a stretchable textile product, preferably a garment, configured to and evidently suitable for forming the wearable electronics product 1 as described herein, in combination with a flexible patch and a securing element, preferably the flexible patch 4 and the securing element 9, as described herein. Preferably the stretchable textile product may comprise a garment selected from: a base layer garment such as a shirt, leggings, short, bra, belt, sleeve, chest band, waist band, arm band, leg band, head band, sock, bandage, and the like

### EXPERIMENTAL RESULTS

### ECG measurements on different conductive skin-contact materials

Experiments were performed to determine the ECG signal quality measured by selected conductive textile electrodes. A commercial gel electrode, specifically a Kendall 133 foam electrode (Fig 10A), was selected as the reference electrode. Each test was performed following the same test protocol, to make results comparable. A pair of electrodes was applied to the skin of the inner forearms of a test subject, with a ground electrode applied at the inner side of the elbow. The ECG signals were recorded and compared to that of the reference electrode. The signal graphs of the different electrode types are shown in Figures 10A to 10F.

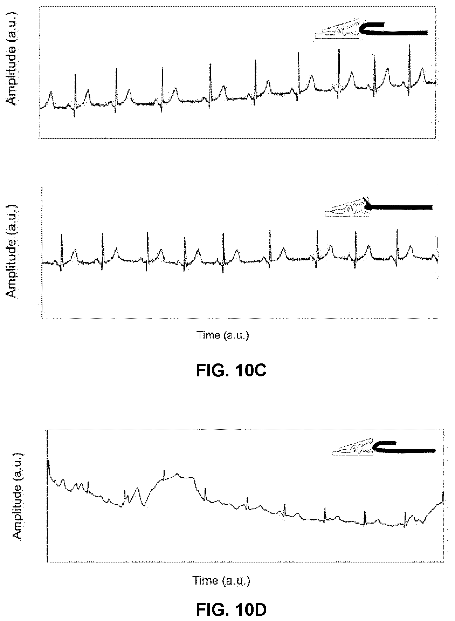

For electrodes of Shieldex ^{®} Technik-tex P130 + B (Fig 10B), and Shieldex ^{®} Silitex P130 (Fig 10C), all ECG peaks were visible in the recorded data (PQRST wave detection), and qualitatively low noise to signal was observed. The signal quality was comparable to that of the reference signal. For electrodes of Shieldex ^{®} Silitex P130 including a silicone coating on the skin facing side (Fig 10D), higher noise to signal was observed, and the signal quality appeared lower compared to that of the reference electrode. This deviation is ascribed to the silicone coating having a lower conductivity than the conductive textile.

### ECG measurements in shirt

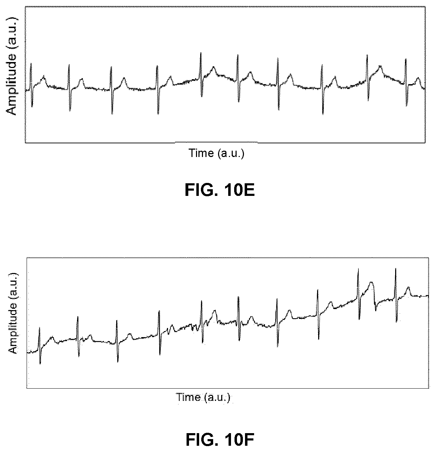

In further tests, the performance of the electrodes were measured when integrated in a textile product, as described herein. Skin contact electrodes comprising conductive textile Shieldex ^{®} Silitex P130 were used. Reference commercial gel electrodes, specifically Kendall 133 foam electrodes, were contacted to the outward surface of the textile skin contact electrodes to measure a first signal (Fig 10E). A printed flexible patch with contact material of silver ink Intexar PE874 DuPont, comprising contact terminals corresponding to the skin contact electrodes, was contacted to the electrodes to measure a second signal (Fig 10F). During measurement, the patch was held in position by a securing element, specifically a pocket, as described herein.

For the setup utilizing the commercial gel electrodes contacting the textile skin contact electrodes, all measured peaks were visible on the ECG graph (P, Q, R, S, T), qualitatively low noise to signal were observed, and the signal quality was comparable to that of the commercial gel electrodes applied directly to the skin.

For the setup utilizing the patch contacted to the textile skin contact electrodes, all measured peaks were visible on the ECG graph (P, Q, R, S, T), qualitatively low noise to signal were observed, and the signal quality was comparable to that of the reference signal. Conclusively, high quality ECG signal was measured for the prototype.

### Stretch measurements of fabrics for shirt and pocket

In order to improve signal quality and stability, as well as comfort during wear, the elasticity and residual strain of the textiles used for the textile product should ideally be similar, preferably matched. Experiments were performed to determine the elasticity and residual strain of several materials for the (i) securing element in the form of a pocket and (ii) the textile skin contact electrodes.

Test samples of materials were subjected to cyclic tensile loads, in a Mark-10 mechanical testing tool. The results of the tests are shown in the table below.

| **Material** | **Textile grain direction** | **E_{eff} cycle 1 (MPa)** | **E_{eff} cycle 3 (MPa)** | **Residual strain (%)** |
|---|---|---|---|---|
| Shieldex ^{®} Silitex P130 | Cross direction | 15.2 | 11.6 | 11 |
| Shieldex ^{®} Silitex P130 | Length direction | 8.4 | 6.5 | 6 |
| Shieldex ^{®} Technik-tex P130 + B | Cross direction | 0.8 | 0.8 | 15 |
| Shieldex ^{®} Technik-tex P130 + B | Length direction | 0.7 | 0.9 | 13 |
| Eurojersey | Cross direction | 5.9 | 5.4 | 14 |
| Eurojersey | Length direction | 2.7 | 2.7 | 11 |
| Compression T-shirt Domyos | Cross direction | 0.7 | 0.6 | 13 |
| Compression T-shirt Domyos | Length direction | 0.6 | 0.5 | 12 |

It was found that Compression T-shirt Domyo material, intended for forming the textile product, and Shieldex ^{®} Technik-tex P130 + B, intended for forming the skin contact electrodes, performed similar during the tests and yielded the most wear comfort. In both the cross-direction and length direction orientations, the aforementioned materials showed an effective Young's modulus (E_{eff}) of below 1 MPa for cycles 1 and 3, as well as a residual strain in a range of 12-15%.

In comparison to the other materials, the textile including silicone Shieldex ^{®} Silitex P130 was significantly stiffer, especially in a cross direction, showing a high effective Young's modulus of 15.2 MPa.

The Eurojersey material is a knitted fabric with an anisotropic knitting direction. This material was stiffer than T-shirt Domyo and Shieldex ^{®} Technik-tex P130 + B in both directions, but more elastic than Shieldex ^{®} Silitex P130.

The residual strain of a material indicates a material's plastic deformation upon stretching, and its ability to retain its original shape after deformation. The test results indicated that all of the materials are comparable in terms of residual strain, all except for Shieldex ^{®} Silitex P130 showing residual strain in a range of 11-15%. In a cross-direction orientation Shieldex ^{®} Silitex P130 showed a value of 11, falling within the range of the other materials. However, in a length direction Shieldex ^{®} Silitex P130 showed a much lower residual strain value of 6%, indicating that it retained its original shape better than the other materials, showing less plastic deformation.

Based on the results of the elasticity and residual strain tests, it was determined that the combination of Compression T-shirt Domyo material, for forming the textile product, and Shieldex ^{®} Technik-tex P130 + B, for forming the skin contact electrodes, would be most suitable for improving wearer comfort, based on similar stretch and deformation properties. When matching elasticity of the shirt material and conductive textile, the difference in materials are less noticeable in comfort since stretchability is comparable.

The elasticity of the Eurojersey material falls in a preferred range of 3-7 times lower than that of the Compression T-shirt Domyo, which makes the Eurojersey a suitable material for forming the pocket-securing element.

For the purpose of clarity and a concise description, features are described herein as part of the same or separate embodiments, however, it will be appreciated that the scope of the invention may include embodiments having combinations of all or some of the features described.

Of course it will be appreciated that the present application can be applied to both human non-human subjects. In practical it is envisioned that the wearable electronics product can be applied to detect/apply bio signals animals including but not limited to mammals such as pets, (sport) horses, cattle, farm animals, etc. Therefore terms as subject, body(parts), wearer, skin, etc., as used herein may similarly apply to animals where appropriate.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. A wearable electronics product (1), comprising
a stretchable textile product (2) for conformable wearing around a body part (3) of a wearer, the stretchable textile product (2) comprising at least one skin contact electrode (5-1) for interfacing with a skin of the body part during wear, the skin contact electrode (5-1) comprising an electrically conductive stretchable textile (15-1) bordered by an electrically insulating stretchable textile (17) , and
a flexible patch (4) supporting thereon an electronic component (42) operably connected to a flexible contact terminal (7-1) of the flexible patch (4),
wherein the wearable electronics product (1) comprises a securing element (9) reversibly mounting the flexible patch (4) along an outward side of the stretchable textile product (2) and applying a contact pressure at a position of an overlap (8-1) between an outward face (35-1) of the skin contact electrode (5-1) and the contact terminal (7-1) forming an electrical interconnect between the skin contact electrode (5-1) and the contact terminal (7-1) at least during wear.

2. The wearable electronics product according to any of the preceding claims, wherein the flexible patch (4) comprises a plurality of contact terminals (7-1,7-2,7-n) and wherein the stretchable textile product (2) comprises at least a corresponding number of skin contact electrodes (5-1,5-2,5-n), whereby
the skin contact electrodes and contact terminals are mutually configured to provide electrical interconnects between the respective contact terminals and separate ones of the skin contact electrodes.

3. The wearable electronics product according to any of the preceding claims, wherein the stretchable textile product (2) comprises a plurality of the skin contact electrodes (5-1,5-2,5-n) each electrically separated from adjacent ones and arranged in an array defining a functional area of the stretchable textile product (2), whereby the skin contact electrodes are dimensioned and interspaced such that the contact terminal (7-1) overlaps with the skin contact electrode (5-1), and any additional contact terminals (7-1,7-2,7-n) overlap with mutually exclusive ones of the skin contact electrodes (5-1,5-2,5-n).

4. The wearable electronics product according to the preceding claim, wherein a maximum cross sectional dimension of each the skin contact electrodes (d5) and a maximum interspacing (s5) between the skin contact electrodes comprised in the array are each smaller than a maximum cross-sectional dimension of the contact terminal (d7) and a minimum interspacing (s7) between adjacent contact terminals, if present.

5. The wearable electronics product according to any of the previous claims, wherein the electronic component (42) comprises one or more selected from the list of a PCB, battery, electronical drive actuator, vibration motor, LED, sensor, I/O devices, and/or photodiode.

6. The wearable electronics product according to any of the previous claims, wherein the securing element (9) has a Young's modulus that is higher than a Young's modulus of the textile product (2).

7. The wearable electronics product according to any of the previous claims, wherein the securing element (9) is a flexible conformable pocket or strap.

8. The wearable electronics product according to any of the previous claims, wherein the electrically conductive stretchable textile (15-1) and the electrically insulating stretchable textile (17) are seamlessly formed as a continuous textile, preferably a woven, nonwoven or knitted textile.

9. The wearable electronics product according to any of the previous claims, wherein the electronic component (42) and any circuitry (10) associated therewith are entirely disposed on the flexible patch (4).

10. The wearable electronics product according to any of the previous claims, wherein securing element (9) is reversibly mountable to the outward side (23) of the stretchable textile product (2).

11. A method (100) of using a wearable electronics product according to any of the preceding claims 1-10, comprising steps of
fitting (101) the stretchable textile product (2) to a body part (3), such that the textile product conformably follows the curvature of the body part; and the functional area of the stretchable textile product (2) covers a region of interest (6) of the body part, known to correspond with positions for receiving a target bio signal of the body part and/or known to correspond with positions for applying an electrical stimulus to the body part; and
reversibly mounting (102) the flexible patch (4) along an outward side (23) of the stretchable textile product (2), within a perimeter of the region of interest, thereby forming an electrical interconnect between respective contact terminals and separate ones of the skin contact electrodes.

12. The method according to claim 11, wherein the method comprises a step of
removably attaching (105) the securing element, preferably after fitting (101), to the outside side of the textile product such that the securing element (9) covers the region of interest (6).

13. A kit of parts (200) for assembling the wearable electronics product (1) according any of claims 1-10, the kit comprising
the stretchable textile product (2) as specified in any of the claims 1-10;
the flexible patch (4) as specified in any of the claims 1-10;
the securing element (9) as specified in any of the claims 1-10; and optional instructions (201) for using the wearable electronics product according to the method specified in claim 11 or 12.

14. A stretchable textile product (2), preferably a garment, configured to and evidently suitable for forming the wearable electronics product (1) according to any of claims 1-10 in combination with an flexible patch and a securing element, preferably the flexible patch (4) and the securing element (9), as specified in any of the claims 1-10.
